# FASCICULE DE BREVET EUROPEEN

(11) **EP 0 627 202 B1**
(45) Date de publication et mention de la délivrance du brevet: **24.06.1998**
(21) Numéro de dépôt: 93420146.8
(22) Date de dépôt: 05.04.1993
(51) Int. Cl.: A61F 2/38

(54) **Ensemble prothétique pour la réalisation d'une prothèse du genou**
Prothesenteile zum Aufbau eines Kniegelenks
Prosthetic assembly for forming a knee joint

(43) Date de publication de la demande: 07.12.1994
(73) Titulaire: PROCOM S.A., 72000 Le Mans (FR)
(72) Inventeur: Almueis, Xavier, F-41260 La Chaussée St Victor (FR); Baudry, Frank, F-49300 Cholet (FR); Bene, Bruno, F-29220 Landerneau (FR); Besse, Jean-Pierre, F-27400 Louviers (FR); Boudieux, Patrick, F-14800 Deauville (FR); Champion, Philippe, F-41100 Vendome (FR); Chata, Georges, F-23000 St Sulpice le Gueretois (FR); Coisy, Michel, F-44230 St Sebastien/Loire (FR); Coppeaux, Jacques, F-86100 Chatellerault (FR); Dosdat, Jean-Claude, F-53000 Laval (FR); Gagna, Gilles, F-72000 Le Mans (FR); Guingand, Olivier, F-75016 Paris (FR); Karren, Christian, F-27025 Evreux (FR); Lazennec, Jean-Yves, F-94000 Creteil (FR); Lebret, Hervé, F-56300 Pontivy (FR); Maignan, Jean-Marc, F-72200 La Flèche (FR); Mairesse, Jean-Louis, F-06000 Nice (FR); Marquer, Yves, F-56000 Vannes (FR); Martinez, Bernard, F-93220 Gagny (FR); De Matteis, Gérard, F-56000 Vannes (FR); Mendolia, Georges, F-62360 Echingen (FR); Meynet, Jean-Claude, F-72000 Le Mans (FR); Pean, Claude, F-72000 Le Mans (FR); Peyrou, Pierre-Louis, F-75007 Paris (FR); De Potter, Jacques, F-95880 Enghien Les Bains (FR); Vernet, Philippe, F-72000 Le Mans (FR); Verola, Jean, F-56000 Vannes (FR); Viale, Patrick, F-18000 Bourges (FR); Zivy, Jean-Noel, F-14123 Fleury/Orne (FR); Cruchet, Patrick, F-72000 Le Mans (FR); Monfort, Jean-Claude, F-72220 Saint Mars D'Outille (FR)
(74) Mandataire: Dupuis, François

(56) Documents cités:
- EP-A- 186 471
- EP-A- 294 298
- EP-A- 346 183
- EP-A- 381 352
- FR-A- 2 663 536
- US-A- 4 892 547

## Description

On connait différents types de prothèses du genou présentant essentiellement un composant fémoral et un composant tibial.

En fonction du cas pathologique à traiter, différentes prothèses sont utilisées. On distingue par exemple, les prothèses tricompartimentaires, bi-compartimentaires et mono-compartimentaire.

Plus particulièrement, l'invention concerne les prothèses tricompartimentales du genou dont le composant fémoral présente deux patins condyliens coopérant en appui et à glissement, avec un plateau tibial solidaire d'une embase. Le composant fémoral peut présenter une tige d'ancrage pour l'impaction au niveau de la tête fémoral du fémur. De même, l'embase tibiale peut présenter une tige d'ancrage pour son impaction dans le tibia. Le plateau tibial est réalisé en polyéthylène et présente des surfaces d'appui interne et externe, profilées en section en fonction du profil des patins condyliens, du composant fémoral.

A partir de cette conception générale de prothèse, il existe sur le marché, de très nombreuses formes de réalisation aptes à résoudre différents problèmes.

On peut citer l'enseignement du brevet FR-A-2663536 qui concerne une prothèse totale du genou du type à glissement. Cette prothèse comprend pour l'essentiel, un élément métallique fixé à l'extrémité inférieure du fémur, un élément métallique fixé à l'extrémité supérieure du tibia, et un élément intermédiaire en matière plastique dont les surfaces en contact avec les éléments tibial et fémoral sont établis de manière complémentaire.

L'élément intermédiaire et l'élément tibial sont agencés avec des moyens de coopération permettant un débattement rotatoire contrôlé limité de l'ensemble élément fémoral - élément intermédiaire, en position de flexion et d'extension de la jambe.

Cependant, quelle que soit la forme de réalisation de ces prothèses, le praticien doit disposer de plusieurs types de prothèses, en fonction du cas pathologique à traiter. Notamment, la prothèse doit être conformée pour tenir compte du maintien ou non des ligaments croisés postérieurs chez le patient.

Le problème que se propose de résoudre l'invention est de pouvoir, à partir d'un même ensemble d'éléments modulaires, traiter différents cas pathologiques.

Pour résoudre un tel problème, d'une part et pour résoudre le problème posé de contrôler la rotation du plateau mobile, il a été conçu et mis au point un ensemble prothétique conforme aux caractéristiques de la revendication 1.

Suivant une autre caractéristique de l'invention, que l'on sélectionne une prothèse à plateau mobile ou à plateau fixe avec conservation des ligaments croisés postérieurs, l'élément fémoral présente des patins condyliens profilés pour générer un contact linéaire et constant, au fur et à mesure de la flexion du genou.

Dans le cas d'une prothèse du genou à plateau fixe avec conservation des ligaments croisés postérieurs, les plateaux fixes sont solidaires de l'embase tibiale par vissage et/ou clipsage.

Pour résoudre le problème posé par l'état osseux, l'élément fémoral et les embases tibiales présentent ou non des tiges d'ancrage.

Pour résoudre le problème posé de toujours avoir un appui linéaire constant, les rayons de courbure des patins condyliens de l'élément fémoral sont identiques deux à deux.

L'invention est exposée, ci-après, plus en détail à l'aide des dessins annexés, dans lesquels :
La figure 1 est une vue de face montrant les principaux éléments prothétiques selon l'invention.
La figure 2 est une vue de dessous du plateau tibial mobile.
La figure 3 est une vue de face de l'élément tibial dans le cas d'un plateau mobile.
La figure 4 est une vue de profil correspondant à la figure 3.
La figure 5 est une vue en coupe, à une échelle plus importante, considérée selon la ligne 5-5 de la figure 3.
La figure 6 est une vue de face de l'élément tibial dans le cas d'un plateau fixe avec conservation des ligaments croisés postérieurs.
La figure 7 est une vue de face de l'élément fémoral apte à coopérer, indiféremment, avec le plateau mobile ou le plateau fixe, avec conservation des ligaments croisés postérieurs.
La figure 8 est une vue de profil correspondant à la figure 7.
La figure 9 est une vue de face d'un élément fémoral conformé pour coopérer avec le plateau fixe, sans conservation des ligaments croisés postérieurs.
La figure 10 est une vue de profil correspondant à la figure 9.
La figure 11 est une vue de face de l'élément tibial dans le cas d'un plateau fixe sans conservation des ligaments croisés postérieurs.
La figure 12 est une vue de profil correspondant à la figure 11.

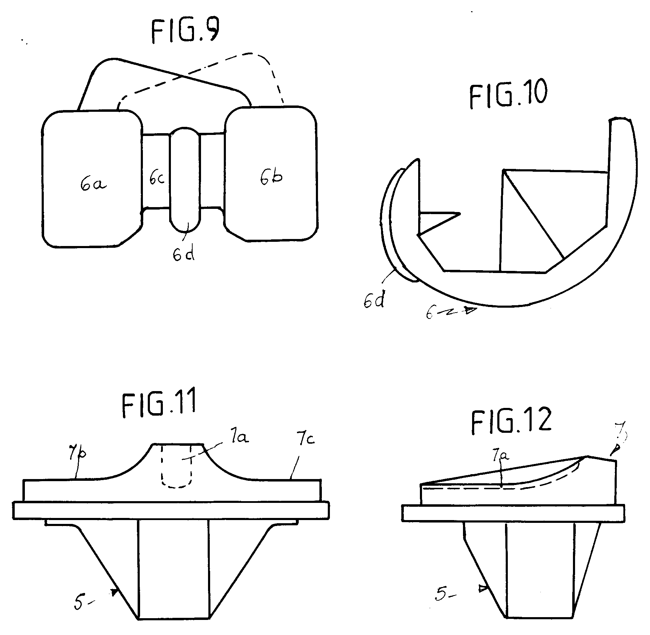

Les figures 9 à 12 ne montrent pas un mode de réalisation selon l'invention.

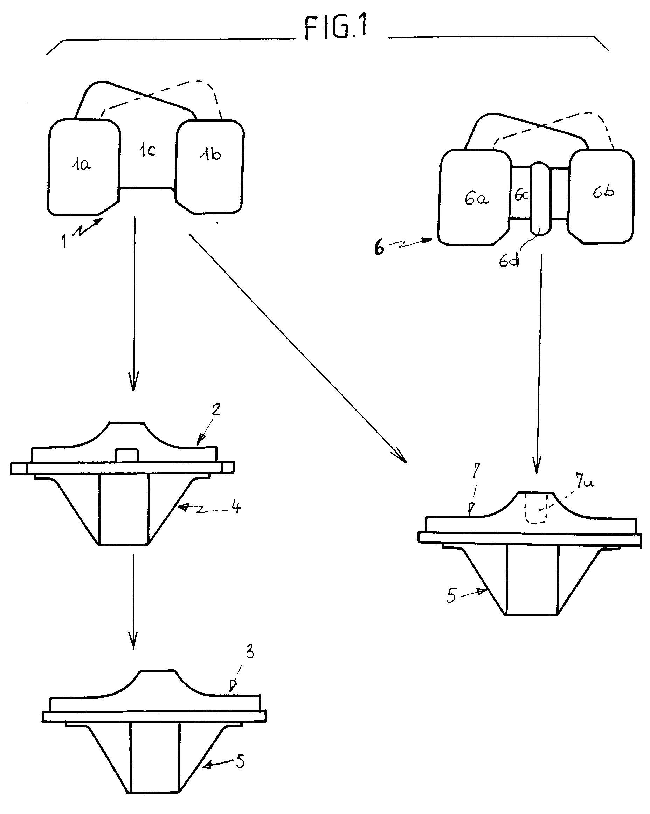

Comme le montre la figure 1, l'ensemble prothétique comprend un élément fémoral désigné dans son ensemble par (1) et apte à coopérer soit avec un plateau mobile (2) d'une embase tibiale (4), soit avec un plateau fixe (3) d'une embase tibiale (5). La figure 1 montre aussi un second type d'élément fémoral désigné dans son ensemble par (6) apte à coopérer avec un plateau fixe (7) de l'embase tibiale ( 5).

Chacun des éléments fémoraux (1) et (6) présente des patins condyliens (1a-1b) et (6a-6b) réunis d'une manière connue par une partie commune (1c-6c) en constituant ou non une trochlée. Le profil externe des patins condyliens est déterminé pour générer sur les plateaux tibiaux correspondants (2) (3) (7), un contact linéaire et constant, au fur et à mesure de la flexion du genou. Les rayons de courbure des patins condyliens sont identiques deux à deux.

D'une manière connue, la face interne des patins condyliens présente tout type d'agencement pour l'impaction de l'élément fémoral, notamment au niveau des condyles du fémur. Par exemple et comme le montrent les figures des dessins, la face interne des patins condyliens (1a-1b) et (6a-6b) présente une série de parts coupés coopérant avec des découpes complémentaires formées au niveau des condyles fémoraux. De même, la face interne des patins condyliens présente, en débordement, des pots d'ancrage ou autres pour l'impaction de cet élément fémoral.

On prévoit également de l'équiper directement ou d'une manière rapportée, d'une tige d'ancrage (8) apte à coopérer, de manière connue, avec la diaphyse du fémur.

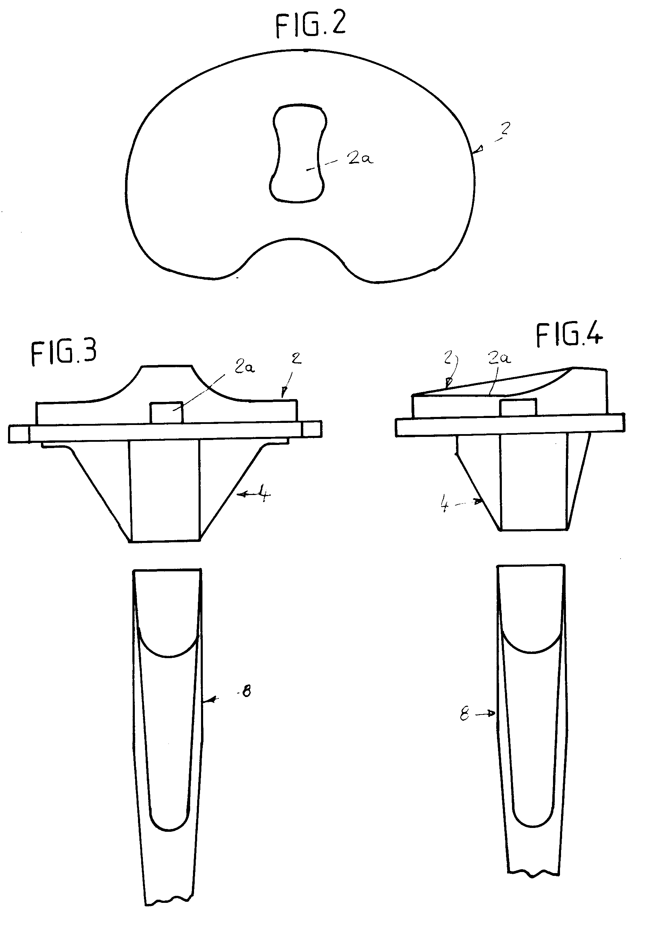

Comme indiqué, l'élément fémoral (1) peut coopérer soit avec un plateau mobile (2), soit avec un plateau fixe (3) (figures 3, 4, 5).

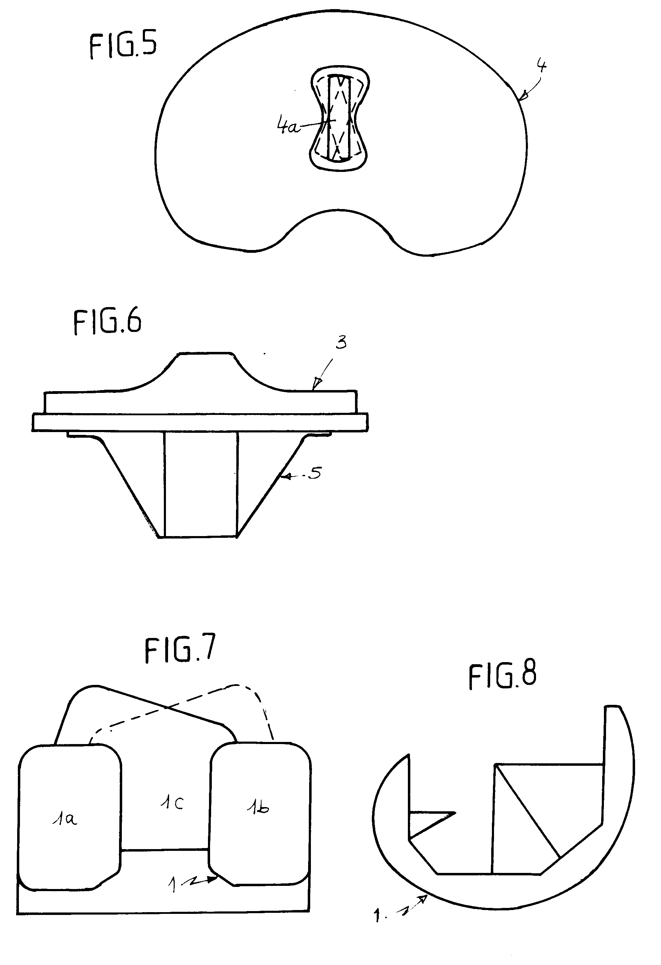

Dans le cas d'un plateau mobile (2), la face de dessous de ce dernier présente dans son épaisseur une empreinte profilée non débouchante (2a), coopérant avec un axe (4a) que présente en débordement, l'embase tibiale (4). Cette empreinte (2a) est profilée très sensiblement en forme de huit ouvert (figure 5). L'axe (4a) est de section rectangulaire, les côtés de plus petite dimension étant arrondis. Après engagement et centrage de l'empreinte (2a) sur l'axe (4a), le plateau (2) peut être soumis à différents mouvements de déplacement de translation et de rotation partielle en notant que le contact en butée et en rotation se fait sur une surface importante.

L'élément fémoral (1) peut également coopérer avec le plateau fixe (3), dans le cas où les ligaments croisés postérieurs sont conservés (figure 5). La fixation du plateau fixe, sur l'embase tibiale (5), peut s'effectuer par tout moyen connu et approprié. Par exemple, le plateau (3) et l'embase tibiale (5), présentent des agencements complémentaires aptes à assurer leur fixation par clipsage. De tels agencements ne sont pas décrits en détail car ils ne font pas partie de l'objet spécifique de l'invention. Eventuellement, cette fixation par clipsage peut être complétée par une fixation à vis.

Dans le cas où il n'est pas possible de conserver les ligaments croisés postérieurs, l'ensemble prothétique comprend un élément fémoral (6) et un plateau tibial (7) (figures 9, 10, 11, 12). Dans ce but et comme le montrent les figures 9 et 10, l'élément fémoral (6) présente entre ces deux patins condyliens (6a) (6b), une prohéminence profilée (6d) sous forme de condyle. Cette prohéminence (6d) est déterminée pour coopérer avec une empreinte en creux complémentaire (7a) formée très sensiblement dans la partie médiane du plateau (7), entre les parties d'appui et de glissement (7b) (7c) recevant les patins condyliens (6a) (6b).

La prohéminence (6d) et l'empreinte (7a) sont dimensionnées pour que ladite prohéminence échappe l'empreinte au fur et à mesure de la flexion du genou, permettant ainsi le mouvement naturel de rotation de l'élément fémoral par rapport à l'élément tibial.

Comme indiqué précédemment, la fixation du plateau (7) sur l'embase tibiale (5) s'effectue par tout moyen connu et approprié, tel que clipsage et/ou vissage.

En ce qui concerne les plateaux tibiaux (2) (3) et (7), ces derniers sont, d'une manière parfaitement connue pour l'homme du métier, réalisés en polyéthylène haute densité et présentent des surfaces d'appui convenablement profilées et de rayon de courbure correspondant au développement du profil des patins condyliens des éléments fémoraux (1) (6). De même, les embases tibiales (4) et (5) recevant respectivement les plateaux (2) (3) et (7), sont exécutées en tout type d'alliage. Ces embases tibiales peuvent présenter directement ou d'une manière rapportée des tiges d'ancrage (9). Dans les exemples illustrés, les embases tibiales reçoivent par vissage, des tigres d'ancrage qui peuvent être de différentes longueurs.

A noter que dans le cas de plateaux fixes, ces derniers s'adaptent de deux en deux :

Par exemple pour des embases tibiales sont de 70 et 75 mm on utilise un plateau de 65 mm.

Dans le cas d'une prothèse à plateau mobile, les rayons de courbure des patins condyliens sont identiques deux à deux :

Par exemple, pour des fémurs 60 et 65 mm, le rayon est R1 tandis que pour des fémurs de 70 et 75, le rayon est R2

Les avantages ressortent bien de la description, en particulier on souligne et on rappelle :
- La possibilité d'obtenir à parti d'un même ensemble prothétique, une prothèse du genou à plateau mobile, une prothèse du genou à plateau fixe avec conservation des ligaments croisés postérieurs.
- Dans le cas d'un plateau mobile, glissement de ce dernier dans tous les plans.
- Elément fémoral adaptable sur plateau tibial, mobile ou fixe.
- Mixage des plateaux polyéthylène sur un même fémur.

## Revendications

1. Ensemble prothétique comprenant un plateau mobile (2) d'une embase tibiale (4) et un élément fémoral (1) apte à coopérer soit avec ledit plateau mobile (2) de ladite embase tibiale (4) pour constituer une prothèse du genou à plateau mobile, soit avec un plateau fixe (3) d'une embase tibiale (5) pour constituer une prothèse du genou à plateau fixe avec conservation des ligaments croisés postérieurs, caractérisé en ce que :
- la face de dessous du plateau mobile (2) présente dans son épaisseur, une empreinte non débouchante (2a),
- l'empreinte (2a) a une forme profilée très sensiblement en huit,
- l'empreinte (2a) coopère avec un axe (4a) que présente en débordement l'embase tibiale (4) pour contrôler le débattement rotatoire et le débattement antéro-postérieur ainsi que médio-latéral du plateau (2).

2. Ensemble selon la revendication 1, caractérisé en ce que l'axe (4a) est de section rectangulaire, les côtés de plus petites dimensions étant arrondis.

3. Ensemble selon la revendication 1, caractérisé en ce que l'élément fémoral (1) présente des patins condyliens (1a-1b) (6a-6b) profilés pour générer un contact linéaire et constant, au fur et à mesure de la flexion du genou.

4. Ensemble selon la revendication 1, caractérisé en ce que l'ensemble prothétique comprend un plateau fixe (3) étant solidaire de l'embase tibiale par vissage et/ou clipsage.

5. Ensemble selon la revendication 1, caractérisé en ce que l'élément fémoral et l'embase tibiale (1) présentent ou non des tiges d'ancrage (9).

6. Ensemble selon la revendication 1, caractérisé en ce que les rayons de courbure des patins condyliens de l'élément fémoral sont identiques deux à deux.

## Claims

1. Prosthetic assembly comprising a mobile plate (2) of a tibial base plate (4) and a femoral element (1) able to co-operate either with said mobile plate (2) of said tibial base plate (4) to form a mobile-plate knee prosthesis or with a fixed plate (3) of a tibial base plate (5) to form a fixed-plate knee prosthesis with conservation of the posterior cruciate ligaments, characterised in that:
- the lower surface of the mobile plate (2) has, within its thickness, a non opening cavity (2a)
- the cavity (2a) has a shape closely resembling a figure of eight,
- the cavity (2a) co-operates with a shaft (4a) that protrudes from the tibial base plate (4) to control the rotatory movement and the antero-posterior and medio-lateral movement of the plate (2).

2. Assembly according to claim 1, characterised in that the shaft (4a) has a rectangular cross-section, the sides of the smallest dimensions being rounded.

3. Assembly according to claim 1, characterised in that the femoral element (1) bears condyle bases (1a-1b) (6a-6b) shaped so as to generate a constant linear contact throughout flexion of the knee.

4. Assembly according to claim 1, characterised in that the prosthetic assembly comprises a fixed plate (3) forming a fixed unit with the tibial base plate by being screwed down and/or by clipage.

5. Assembly according to claim 1, characterised in that the femoral element and the tibial base plate (1) bear or do not bear anchoring shafts (9).

6. Assembly according to claim 1, characterised in that the corresponding radii of the curvature of the condyle bases of the femoral element are identical.

## Patentansprüche

1. Prothesensystem bestehend aus einem beweglichen Plateau (2) eines Tibiabasisplateaus (4) und einem Femurelement (1), das geeignet ist, entweder mit dem gesagten beweglichen Plateau (2) des gesagten Tibiabasisplateaus (4) zwecks Bildung einer Knieprothese mit mobilem Plateau oder mit einem ortsfesten Plateau (3) eines Tibiabasisplateaus (5) zur Bildung einer Knieprothese mit ortsfestem Plateau bei Konservierung der hinteren Kreuzbänder zusammenzuwirken, dadurch gekennzeichnet, daß:
- die Unterseite des beweglichen Plateaus (2) in der Materialdicke eine nicht nach außen führende Vertiefung (2a) aufweist,
- die Vertiefung (2a) eine ziemlich genau achterförmige Profilform besitzt,
- die Vertiefung (2a) mit einer Achse (4a) zusammenwirkt, die aus dem Tibiabasisplateau (4) herausragt, um die Rotationsbewegung, die anteroposteriore Bewegung und die mediolaterale Bewegung des Plateaus (2) zu kontrollieren.

2. Prothesensystem nach Anspruch 1, dadurch gekennzeichnet, daß die Achse (4a) einen rechtwinkligen Querschnitt besitzt, wobei die kürzeren Seitenteile abgerundet sind.

3. Prothesensystem nach Anspruch 1, dadurch gekennzeichnet, daß das Femurelement (1) profilierte Kondylenschuhe (1a-1b) (6a-6b) aufweist, um nach Maßgabe der Kniebeugung einen linearen und konstanten Kontakt zu bewirken.

4. Prothesensystem nach Anspruch 1, dadurch gekennzeichnet, daß die Prothese ein ortsfestes Plateau (3) besitzt, das durch Verschrauben und/oder Aufschnappen mit dem Tibiabasisplateau fest verbunden ist.

5. Prothesensystem nach Anspruch 1, dadurch gekennzeichnet, daß das Femurelement und das Tibiabasisplateau (1) Verankerungsschäfte (9) aufweisen oder nicht.

6. Prothesensystem nach Anspruch 1, dadurch gekennzeichnet, daß die Krümmungsradien der Kondylenschuhe des Femurelements paarweise identisch sind.
